# EUROPEAN PATENT SPECIFICATION

(11) **EP 2 868 657 B1**
(45) Date of publication and mention of the grant of the patent: **30.08.2017**
(21) Application number: 12881905.9
(22) Date of filing: 13.12.2012
(51) Int. Cl.: C07D 311/30

(54) **METHOD FOR PREPARING HIGH PURITY SCUTELLARIN AGLYCONE**
VERFAHREN ZUR HERSTELLUNG VON HOCHREINEM SCUTELLARIN-AGLYCON
PROCÉDÉ DE PRÉPARATION D'AGLYCONE DE SCUTELLARINE À HAUTE PURETÉ

(30) Priority: 23.07.2012 CN 201210256030
(43) Date of publication of application: 06.05.2015
(73) Proprietor: KPC Pharmaceuticals, Inc., Kunming, Yunnan 650106 (CN)
(72) Inventor: ZHANG, Wei, Kunming Yunnan 650106 (CN); LI, Penghui, Kunming Yunnan 650106 (CN); YANG, Zhaoxiang, Kunming Yunnan 650106 (CN); ZHANG, Xiongbo, Kunming Yunnan 650106 (CN); ZHU, Haibei, Kunming Yunnan 650106 (CN); WANG, Jun, Kunming Yunnan 650106 (CN); CHEN, Jinxin, Kunming Yunnan 650106 (CN)
(74) Representative: Bösl, Raphael Konrad
(86) International application number: PCT/CN2012/086545
(87) International publication number: WO 2014/015625

(56) References cited:
- CN-A- 101 684 112
- CN-A- 101 941 999
- JIAN-MEI CUI ET AL: "Total synthesis of scutellarin-7- O -Glucuronide", JOURNAL OF ASIAN NATURAL PRODUCTS RESEARCH, vol. 7, no. 4, 1 August 2005 (2005-08-01), pages 655-660, XP055183994, ISSN: 1028-6020, DOI: 10.1080/1028602032000169587
- RANGASWAMI S ET AL: "Anthoxanthin pigments of the leaves of Digitalis lanata EHRH and the constitution of a new pigment dinatin", PROCEEDINGS OF THE INDIAN ACADEMY OF SCIENCES. SECTION A PHYSICAL SCIENCES, INDIAN ACADEMY OF SCIENCES, IN, vol. 54A, 1 January 1961 (1961-01-01), pages 51-59, XP009187232, ISSN: 0370-0089
- CUI, JIANMEI ET AL.: 'Total synthesis of scutellarin-7-O-Glucuronide' JOURNAL OF ASIAN NATURAL PRODUCTS RESEARCH vol. 7, no. 4, August 2005, pages 655 - 660, XP055183994

## Description

### FIELD OF THE INVENTION

The present invention relates to the field of pharmaceutical synthesis, and particularly, to a method for preparing scutellarin aglycone.

### BACKGROUND OF THE INVENTION

Scutellarin aglycone (CAS: 529-53-3, molecular formula: C₁₅H₁₀O₆, and molecular weight: 286.24) has the structural formula of Formula (I):

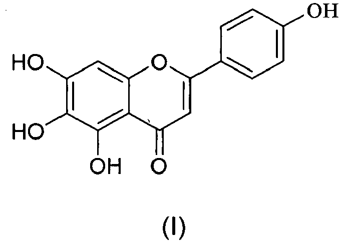

Scutellarin has very definite therapeutic effect on ischemic cerebrovascular disease, coronary heart diseases, hypertension, cerebral hemorrhage, cerebral thrombosis, cerebral embolism polyneuritis, chronic arachnitis and the sequelae thereof, in which scutellarin plays its therapeutic effect through scutellarin aglycone. Recent pharmacological studies have revealed that the in vivo active metabolites of scutellarin include scutellarin aglycone and the glucuronidated, sulfated and methylated conjugates of the aglycone.

Scutellarin can be synthesized by acylation hydroxy protection, glycosylation and deacetylation of scutellarin aglycone. A semi-synthetic method has been disclosed for preparing scutellarin aglycone by hydrolysis of scutellarin, followed by de-glycosylation in application Nos. 200410033687.5, 200910087672.x and 200910087673.4.

The existing references and public patents regarding the preparation of scutellarin by chemical synthesis include: "A method for preparing 5,6,4'-trihydroxy-flavonoid-7-O-D-glucuronic acid" (CN101538298A, CN101538299A) to He Zhengyou and Liu Junyan *et al.*; "A method for preparing scutellarin" (CN101941999A) to Yang Jian *et al.*; Cui Jianmei et al., Total synthesis of scutellarin - 7 - O - Glucuronide, Journal of Asian Natural Products Research 2005, 7(4):655-660; A method related to the chemical synthesis of scutellarin aglycone was disclosed in Cui Jianmei et al., Natural Product Research and Development, 2003, 15, 3, 255-258. However, the method mentioned obtained only an intermediate, scutellarin aglycone, with purity of 60-85%, and the impurities generated had very similar chromatographic behavior as scutellarin aglycone. Besides, the physicochemical properties of scutellarin aglycone were in fact very unstable with poor solubility, so that it was difficult to obtain scutellarin aglycone with high purity (content greater than 98%) simply by recrystallization or column chromatography under basic and strong oxidizing conditions.

Based on the facts mentioned above, the reports on the industrial synthesis of scutellarin aglycone with high purity are not favorable for commercial process.

### SUMMARY OF THE INVENTION

A novel simple method, which can be used in the commercial process, for preparing scutellarin aglycone with high yield and high purity is provided in the present invention, in order to address the disadvantages of the methods in the prior art for preparation of scutellarin aglycone, by which scutellarin aglycone with low purity was obtained, and which was difficult for application in the commercial process.

5,6,7,4'-tetramethoxy flavonoid having a structure of Formula (III) was demethylated to give a crude product of scutellarin aglycone according to Step 1 of claim 1, which was protected by an acetylated hydroxy group to obtain a compound having a structure of Formula (II) according to Step 2 of claim 1. Subsequently, a scutellarin aglycone product was obtained for further purifying after acidic or basic hydrolysis of the compound of Formula (II) according to Step 3 of claim 1. By simple purifying, scutellarin aglycone of Formula (I) was obtained with high purity according to Step 4 of claim 1. The method of the invention is further characterized in that
(a) the solvent in step (1) is selected from one or more of dichloromethane, trichloromethane, dimethyl sulfoxide, dimethyl formamide and pyridine;
(b) the demethylation reagent in step (1) is boron tribromide or anhydrous aluminium trichloride;
(c) in step (1), the reaction temperature is in the range from 0 to 30°C when boron tribromide is used as the demethylation reagent, and in the range from 30 to 200°C when anhydrous aluminium trichloride is used as the demethylation reagent;
(d) the temperature for the hydrolysis reaction in step (3) is from -10°C to 40°C, and the reaction duration is from 1 to 20 hours; and
(e) the reaction solvent in step (3) is selected from one or more of acetone, water, methanol, ethanol, dichloromethane, dimethyl sulfoxide, tetrahydrofuran or dioxane.

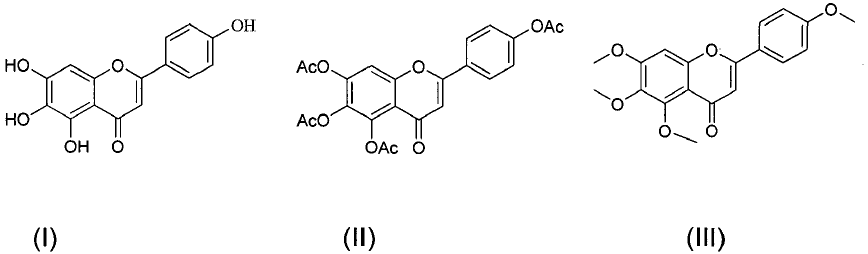

The present invention has the advantages, such as simple procedure, mild reaction conditions, cheap reagents, no ring-opening isomerization of flavonoid nucleus due to high temperature during demethylation, and difficulties in the following separation and purification process. Scutellarin aglycone obtained with high purity, and easy scale-up for commercial process etc., which comprises the following steps:
Step 1: 5,6,7,4'-tetramethoxy flavonoid of Formula (III) was demethylated in a solvent by a demethylation reagent, to obtain a crude product of compound of Formula (I) with purity of 50%-95%;
   The reaction solvent is selected from one or more of dichloromethane, trichloromethane, dimethyl sulfoxide, dimethyl formamide and pyridine, and the demethylation reagent is boron tribromide or anhydrous aluminium trichloride. The reaction temperature is controlled in the range from 0 to 30°C when boron tribromide is used as the demethylation reagent, and in the range from 30 to 200°C when anhydrous aluminium trichloride is used as the demethylation reagent. Preferably, the reaction duration for step (1) is from 3 to 20 hours. Further preferred is the usage of demethylation reagent in a molar ratio from 1:1 to 50:1 with respect to 5,6,7,4'-tetramethoxy flavonoid.
Step 2: the crude product of compound of Formula (I) was refluxed with acetic anhydride for acylation when pyridine is used as the solvent and catalyst, in order to obtain the compound of Formula (II).
Step 3: the compound of Formula (II) was hydrolyzed by acid or alkali to obtain the compound of Formula (I) for further purifying, whereas the temperature for the hydrolysis reaction is from -10°C to 40°C with the reaction duration from 1 to 20 hours and the reaction solvent is selected from one or more of acetone, water, methanol, ethanol, dichloromethane, dimethyl sulfoxide, tetrahydrofuran or dioxane.
   The alkali used is preferably selected from one or more of sodium hydroxide, lithium hydroxide, potassium hydroxide, potassium carbonate, or sodium carbonate, and its usage is in a molar ratio from about 0.1:1 to 50:1 with respect to the compound (II).
   The acid used is selected from one or two of sulphuric acid and hydrochloric acid, and its usage is in a molar ratio from about 0.1:1 to 50:1 with respect to the compound (II).
Step 4: the compound of Formula (I) for further purifying is refluxed with acetic acid using an aliphatic alcohol having 1 to 3 carbon atoms as the solvent. After filtration, the press cake is washed using the corresponding aliphatic alcohol. After dried under reduced pressure, scutellarin aglycone is obtained with high purity.

Preferably, the amount of acetic acid added is in the volume percent of 0- 10% based on the volume of the aliphatic alcohol, and the duration for reflux is in the range from 1 to 10 hours. The drying under reduced pressure was carried out at 60°C or below.

As compared with the processes in the prior art, the preparing method for scutellarin aglycone described herein has the following advantages:
(1) The ring-opening isomerization of flavonoid nucleus due to high temperature during demethylation and the difficulties in the following separation and purification process are avoided.
(2) Low cost industrial chemicals or medical intermediate are employed for the complete synthesis of scutellarin aglycone with high-purity, and the process can be used for commercial process.
(3) The crude product of scutellarin aglycone is protected by acetylated hydroxy group, which enhanced the polarity of both impurity and acetylated scutellarin aglycone (II), so that the acetylated scutellarin aglycone can be obtained with high purity simply by recrystallization. After hydrolysis of acetylated scutellarin aglycone with high purity by acid or alkali, scutellarin can be obtained with high purity.
(4) The problem of extremely low yield of scutellarin aglycone with high-purity obtained by direct recrystallization after demethylation of (III) was thoroughly solved, and a total yield over 60% can be obtained by the new process.

### BRIEF DESCRIPTION OF THE FIGURE

Fig. 1 is the HPLC chromatogram of the crude product of scutellarin aglycone.
Fig. 2 is the HPLC chromatogram of scutellarin aglycone with high purity described herein.

### DETAILED DESCRIPTION OF THE INVENTION

In order to further understanding the present invention, preferred embodiments of the present invention were described in combination with the following examples, however, it should be appreciated that these descriptions were only used to further illustrate the characteristics and advantages of the present invention, but not construed to limit the claims of the present invention.

A method for preparation of scutellarin aglycone is disclosed by the present invention, which can be implemented by properly modifying the processing parameters by the one of skill in the art with reference to the content herein. Particularly, it should be noted that all similar replacements and modifications are apparent to the one of skill in the art, all of which are regarded to be included in the present invention. The method of the present invention has been described by preferred Examples, and it is apparent that modification, or proper change and the combination thereof can be made to the method and the application described herein by those skilled in the art, without departing from the content and scope of the claimed invention, in order to achieve and apply the techniques disclosed in the present invention.

The present invention will be further explained with reference to the specific Examples below, in order to make the technical proposal of the present invention better understood by one of ordinary skill in the art. The preparation method for scutellarin aglycone will be specifically described below, wherein the compounds having the structure of Formula (I) to (III) are referred as compound (I) to (III) for short.

### Preparative Examples

### 1. Preparation of the crude product of compound (I)

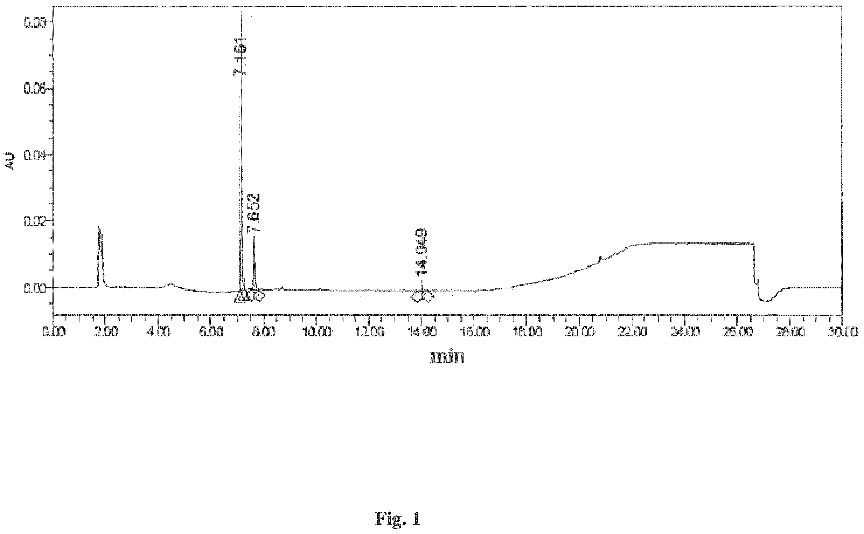

15.0g compound (III) was dissolved in 300 mL trichloromethane, and the temperature for the reaction system was cooled to about 0 °C. The mixed solution of boron tribromide (21 mL) and trichloromethane (150 mL) was slowly added dropwise under stirring, during which the inside temperature was maintained below 30 °C. After the addition, the solution was stirred over night (about 18 hours). The temperature of the reaction system was reduced to about 15 °C on next day. The reaction was quenched by slowly adding 50 mL water dropwise, and the temperature of the reaction system was maintained from 15 to 25 °C. After quenching, another 400 mL water was added to the system for dilution. After the addition of water, the temperature increased to room temperature naturally, and the stirring was maintained for another 1 hour. After filtration (or centrifugation), yellow viscous crude product was obtained. After drying, 12.4 g crude product of compound (I) was obtained (See Fig. 1 for the chromatogram) with purity of 85.0% and yield of 98.4%.

### 2. Preparation of the crude product of compound (I)

After adding 290 g anhydrous aluminium trichloride to 400 mL pyridine, the temperature was enhanced to 120 °C. Subsequently, 15.0 g compound (III) was added and the mixture was stirred for 45 min. The reaction was then stopped. After the reaction temperature was cooled down to 90 °C, the reactant solution was poured into 250 ml mixed solution of water: methanol (1:1). After stirring for another 1 hour, the mixture was filtered, and the press cake was washed by 40 mL ethanol. After drying, 10.0 g crude product of compound (I) was obtained (chromatographical purity of 82.0%) with yield of 65.0%.

### 3. Preparation of the crude product of compound (I)

15.0 g compound (III) was dissolved in 500 mL dichloromethane, and the temperature for the reaction system was cooled to about 4 °C. The mixed solution of boron tribromide (15 mL) and dichloromethane (200 mL) was slowly added dropwise under stirring, during which the inside temperature was maintained at 0 °C. After the addition, the solution was stirred over night (about 20 hours). The temperature of the reaction system reached 15 °C on next day. The reaction was quenched by slowly adding 40 mL water dropwise, and the temperature of the reaction system was maintained from 15 to 25 °C. After quenching, another 400 mL water was added to the system for dilution. After the addition of water, the temperature increased to room temperature naturally, and the stirring was maintained for another 1 hour. After filtration (or centrifugation), yellow viscous crude product was obtained. After drying, 13 g crude product of compound (I) was obtained (chromatographical purity of 84.0%) with yield of 99%.

### 4. Preparation of the crude product of compound (I)

After adding 290 g anhydrous aluminium trichloride to 400 mL dimethyl formamide, the temperature was enhanced to 190 °C. Subsequently, 15.0 g compound (III) was added and the mixture was stirred for 30 min. The reaction was then stopped. After the reaction temperature was cooled down to 50 °C, the reactant solution was poured into 300 ml water. After stirring for about another 2 hours, the mixture was filtered, and the press cake was washed by 40 mL methanol. After drying, 9.0 g crude product of compound (I) was obtained (chromatographical purity of 90.0%) with yield of 70.0%.

### 5. Preparation of compound (II)

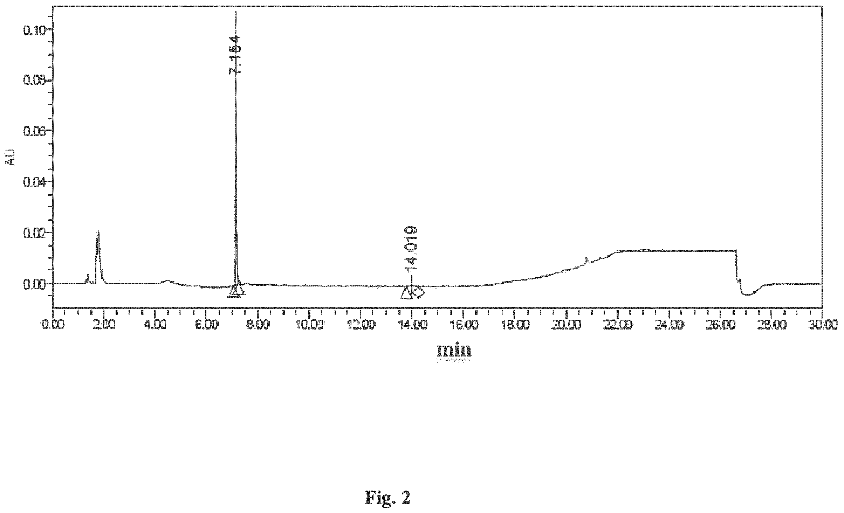

10 g compound (I) was dissolved in 70 ml pyridine, into which 50 mL acetic anhydride was slowly added at room temperature under stirring. After reflux for 9 hours by heating to 120°C, the reaction was confirmed for completion by TLC. When the temperature of the reaction solution was cooled down to 70°C, 90 ml ethyl acetate was added, with a great amount of white solid precipitated gradually. The mixture was stirred for another 1 hour and then cooled to room temperature. Subsequently, it was stood over night in a refrigerator. After filtration, off-white solid was obtained. After drying the press cake, 13 g off-white compound (II) was obtained by recrystallization using ethanol (See Fig. 2 for the chromatogram), with a chromatographical purity over 90.0% and yield of 78%.

¹H NMR (400 MHz, CDCl₃) δ ppm: 7.86-7.88 (d, J = 8.8 Hz, 2H, H-a and a'), 7.25-7.27(d, J = 6.0 Hz, 2H, H-b and b'), 7.49 (s, 1H, H-A), 6.62 (s, 1H, H-B), 2.34∼2.44(s, 12H, 4CH₃).

### 6. Preparation of the compound (I) with high-purity described herein

A mixed solvent of 100 ml acetone and 100 ml water was added to 4.5 g (10 mmol) compound (II). Subsequently, 85 ml 10% aqueous potassium hydroxide solution was slowly added dropwise at -10°C. After stirring for about 2 hours, the reaction was confirmed for completion by TLC. The reaction solution was neutralized to pH 2-3 by slowly adding 2 mol/L aqueous hydrochloric acid solution dropwise to the reaction solution at -7°C. After continuous stirring for 1 hour, the temperature increased gradually to room temperature. Subsequently, yellow solid precipitated gradually in the reaction solution. After standing at room temperature over night, the mixture was filtered, and the press cake was washed by an appropriate amount of water and acetone. After drying, 2.7 g compound (I) with high-purity (chromatographical purity of 98.7%) was obtained with yield of 95% by recrystallization using 1% acetic acid solution in ethanol.

¹H NMR (400 MHz, DMSO-d₆) δ ppm: 7.88-7.90 (d, J = 8.8 Hz, 2H, H-a and a'), 6.88-6.90(d, J = 8.8 Hz, 2H, H-b and b'), 6.73 (s, 1H, H-A), 6.55 (s, 1H, H-B).

### 7. Preparation of the compound (I) with high-purity described herein

80 ml dichloromethane was added to 4.5 g (10 mmol) compound (II). Subsequently, 10 ml 1 mol/L aqueous sodium hydroxide solution was slowly added dropwise at 0°C. After stirring for about 3 hours, the reaction was confirmed for completion by TLC. The reaction solution was neutralized to pH 2-3 by slowly adding 2 mol/L aqueous hydrochloric acid solution dropwise to the reaction solution at 0°C. After continuous stirring for 1 hour, the temperature increased gradually to room temperature. Subsequently, yellow solid precipitated gradually in the reaction solution. After standing at room temperature over night, the mixture was filtered, and the press cake was washed by an appropriate amount of water and acetone. After drying, 3.0 g compound (I) with high-purity (chromatographical purity of 98.0%) was obtained with yield of 97% by recrystallization using 10% acetic acid solution in ethanol.

### 8. Preparation of the compound (I) with high-purity described herein

50 ml dimethyl formamide was added to 4.5 g (10 mmol) compound (II). Subsequently, 40 ml 2 mol/L aqueous lithium hydroxide solution was slowly added dropwise at -4°C. After stirring for about 4 hours, the reaction was confirmed for completion by TLC. The reaction solution was neutralized to pH 2-3 by slowly adding 1 mol/L aqueous hydrochloric acid solution dropwise to the reaction solution at 0°C. After continuous stirring for 1 hour, the temperature increased gradually to room temperature. Subsequently, yellow solid precipitated gradually in the reaction solution. After standing at room temperature over night, the mixture was filtered, and the press cake was washed by an appropriate amount of water and acetone. After drying, 2.5 g compound (I) with high-purity (chromatographical purity of 98.3%) was obtained with yield of 95% by recrystallization using ethanol.

### 9. Preparation of the compound (I) with high-purity described herein

64 g compound (II) was suspended in 1000 mL absolute methanol, into which 10 mL concentrated sulfuric acid was added dropwise under stirring. The mixture was refluxed by heating for about 3 hours until the reaction was completed. The reflux was maintained over night (20 hours), during which the reaction solution changed from milk white gradually to yellow, and the solid was initially dissolved and then a great amount of yellow solid precipitated. On the next day, the stirring and heating were stopped, and the mixture was cooled to room temperature. After standing for 5 hours at room temperature, it was filtered and the press cake was washed with 200 mL absolute methanol. After drying, 37 g yellow compound (I) with high-purity (chromatographical purity of about 99.0%) was obtained with yield of 92.3%.

### 10. Preparation of the compound (I) with high-purity described herein

64 g compound (II) was suspended in 700 mL anhydrous alcohol, into which 20 mL concentrated hydrochloric acid was added dropwise under stirring. The reaction system was heated to reflux. The reflux was maintained over night (20 hours), during which the reaction solution changed from milk white gradually to yellow, and the solid was initially dissolved and then a great amount of yellow solid precipitated. On the next day, the stirring and heating were stopped, and the mixture was cooled to room temperature. After standing for 5 hours at room temperature, it was filtered and the press cake was washed with 300 mL anhydrous alcohol. After drying, 36 g yellow compound (I) with high-purity (chromatographical purity of about 98.7%) was obtained with yield of 91.7% by recrystallization using 10% acetic acid solution in ethanol.

### 11. Preparation of the compound (I) with high-purity described herein

64 g compound (II) was suspended in 500 mL isopropanol, into which 10 mL concentrated sulfuric acid was added dropwise under stirring. The reaction system was heated to reflux. The reflux was maintained over night (20 hours), during which the reaction solution changed from milk white gradually to yellow, and the solid was initially dissolved and then a great amount of yellow solid precipitated. On the next day, the stirring and heating were stopped, and the mixture was cooled to room temperature. After standing for 5 hours at room temperature, it was filtered and the press cake was washed with 100 mL isopropanol. After drying, 40 g yellow compound (I) with high-purity (chromatographical purity of about 98.8%) was obtained with yield of 94% by recrystallization using 2% acetic acid solution in isopropanol.

## Claims

1. A method for preparing scutellarin aglycone with high purity, comprising the following steps:
Step 1: 5,6,7,4'-tetramethoxy flavonoid of Formula (III) was demethylated in a solvent by a demethylation reagent, to obtain a crude product of compound of Formula (I);
Step 2: the crude product of compound of Formula (I) was refluxed by heating with acetic anhydride for acylation when pyridine is used as the solvent and catalyst, in order to obtain the compound of Formula (II);
Step 3: the compound of Formula (II) was hydrolyzed by acid or alkali to obtain the compound of Formula (I) for further purifying;
Step 4: the compound of Formula (I) for further purifying is refluxed with acetic acid using an aliphatic alcohol having 1 to 3 carbon atoms as the solvent, and after filtration, the press cake is washed using the corresponding aliphatic alcohol, and dried under reduced pressure, to obtain scutellarin aglycone with high purity; 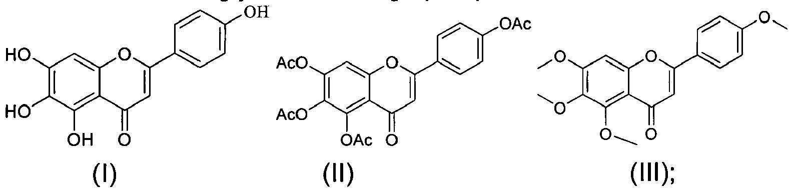
**characterized in that**
(a) the solvent in step (1) is selected from one or more of dichloromethane, trichloromethane, dimethyl sulfoxide, dimethyl formamide and pyridine;
(b) the demethylation reagent in step (1) is boron tribromide or anhydrous aluminium trichloride;
(c) in step (1), the reaction temperature is in the range from 0 to 30°C when boron tribromide is used as the demethylation reagent, and in the range from 30 to 200°C when anhydrous aluminium trichloride is used as the demethylation reagent;
(d) the temperature for the hydrolysis reaction in step (3) is from -10°C to 40°C, and the reaction duration is from 1 to 20 hours; and
(e) the reaction solvent in step (3) is selected from one or more of acetone, water, methanol, ethanol, dichloromethane, dimethyl sulfoxide, tetrahydrofuran or dioxane.

2. The method according to claim 1, which is **characterized in that** the usage of the demethylation reagent in step (1) is in a molar ratio from 1:1 to 50:1 with respect to 5,6,7,4'-tetramethoxy flavonoid, and the reaction duration is from 3 to 20 hours.

3. The method according to claim 1, which is **characterized in that** the alkali used in step (3) is selected from one or more of sodium hydroxide, lithium hydroxide, potassium hydroxide, potassium carbonate, or sodium carbonate, and its usage is in a molar ratio from 0.1:1 to 50:1 with respect to the compound (II).

4. The method according to claim 1, which is **characterized in that** the acid used in step (3) is selected from one or two of sulphuric acid and hydrochloric acid, and its usage is in a molar ratio from 0.1:1 to 50:1 with respect to the compound (II).

5. The method according to claim 1, which is **characterized in that** the duration for reflux in step (4) is in the range from 1 to 10 hours.

## Patentansprüche

1. Verfahren zum Herstellen von hochreinem Skutellarin-Aglykon umfassend die folgenden Schritte:
Schritt 1: 5,6,7,4'-Tetramethoxy-Flavonoid der Formel (III) wurde in einem Lösungsmittel durch ein Demethylierungs-Reagens demethyliert, um ein Rohprodukt der Verbindung von Formel (I) zu erhalten;
Schritt 2: das Rohprodukt der Verbindung der Formel (I) wurde durch Erhitzen mit Essigsäureanhydrid für die Acylierung refluxiert, wenn Pyridin als Lösungsmittel und Katalysator verwendet wird, um die Verbindung der Formel (II) zu erhalten;
Schritt 3: Die Verbindung der Formel (II) wurde durch Säure oder Alkali hydrolysiert um die Verbindung der Formel (I) für die weitere Reinigung zu erhalten;
Schritt 4 : Die Verbindung der Formel (I) für die weitere Reinigung wurde mit Essigsäure unter Verwendung eines aliphatischen Alkohols mit 1 bis 3 Kohlenstoffatomen als das Lösungsmittel refluxiert und nach Filtration wurde der Presskuchen unter Verwendung des korrespondierenden aliphatischen Alkohols gewaschen und unter vermindertem Druck getrocknet, um hochreines Skutellarin-Aglykon zu erhalten; 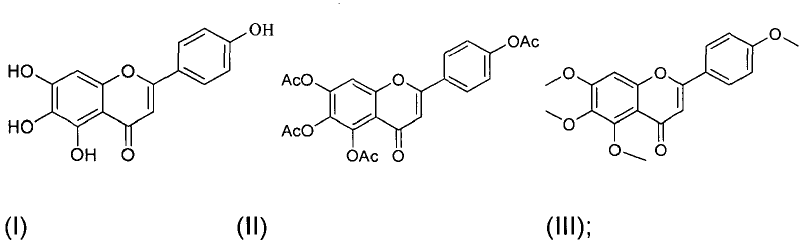
**dadurch gekennzeichnet, dass**
a) das Lösungsmittel in Schritt (1) ausgewählt ist aus einem oder mehreren von Dichlormethan,Trichlormethan, Dimethylsulfoxid, Dimethylformamid und Pyridin;
b) das Demethylierungs-Reagens in Schritt (1) Bortribromid oder wasserfreies Aluminiumtrichlorid ist;
c) in Schritt (1) die Reaktionstemperatur in dem Bereich von 0 bis 30° C ist, wenn Bortribromid verwendet wird als Demethylierungs-Reagens und in dem Bereich von 30 bis 200° C ist, wenn wasserfreies Aluminiumtrichlorid als das Demethylierungs-Reagens verwendet wird;
d) die Temperatur für die Hydrolysereaktion in Schritt (3) von -10° C bis 40° C ist, und die Reaktionsdauer von 1 bis 20 Stunden ist; und
e) das Reaktionslösungsmittel in Schritt (3) ausgewählt ist aus einem oder mehreren von Aceton, Wasser, Methanol, Ethanol, Dichlormethan, Dimethylsulfoxid, Tetrahydrofuran oder Dioxan.

2. Verfahren nach Anspruch 1, **dadurch gekennzeichnet, dass** die Verwendung des Demethylierungs-Reagens in Schritt (1) in einem molaren Verhältnis von 1:1 bis 50:1 im Hinblick auf 5,6,7,4'-Tetramethoxyflavonoid ist und die Reaktionsdauer von 3 bis 20 Stunden ist.

3. Verfahren nach Anspruch 1, **dadurch gekennzeichnet, dass** das Alkali, das in Schritt (3) verwendet wird, ausgewählt ist aus einem oder mehreren von Natriumhydroxid, Lithiumhydroxid, Kaliumhydroxid, Kaliumcarbonat oder Natriumcarbonat und seine Verwendung in einem molaren Verhältnis von 0,1:1 zu 50:1 im Hinblick auf Verbindung (II) ist.

4. Verfahren nach Anspruch 1, **dadurch gekennzeichnet, dass** die Säure, die in Schritt (3) verwendet wird, ausgewählt ist aus einem oder zwei von Schwefelsäure und Salzsäure, und ihre Verwendung in einem molaren Verhältnis von 0,1:1 bis 50:1 im Hinblick auf die Verbindung (II) ist.

5. Verfahren nach Anspruch 1, **dadurch gekennzeichnet, dass** die Dauer des Refluxierens in Schritt (4) im Bereich von 1 bis 10 Stunden ist.

## Revendications

1. Procédé de préparation d'aglycone de scutellarine avec une pureté élevée, comprenant les étapes suivantes :
étape 1 : du 5,6,7,4'-tétraméthoxy-flavonoïde de formule (III) a été déméthylé dans un solvant par un réactif de déméthylation, pour obtenir un produit brut du composé de formule (I) ;
étape 2 : le produit brut du composé de formule (I) a été passé au reflux par chauffage avec de l'anhydride acétique pour une acylation quand la pyridine est utilisée comme solvant et catalyseur, pour obtenir le composé de formule (II) ;
étape 3 : le composé de formule (II) a été hydrolysé par un acide ou un produit alcalin pour obtenir le composé de formule (I) pour une autre purification ;
étape 4 : le composé de formule (I) pour une autre purification est passé au reflux avec de l'acide acétique en utilisant un alcool aliphatique ayant 1 à 3 atomes de carbone comme solvant et, après filtration, le tourteau est lavé en utilisant l'alcool aliphatique correspondant, et séché sous pression réduite, pour obtenir l'aglycone de scutellarine avec une pureté élevée ; 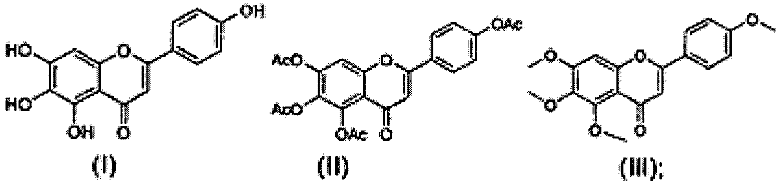 **caractérisé en ce que** :
(a) le solvant dans l'étape (1) est choisi parmi un ou plusieurs du dichlorométhane, du trichlorométhane, du diméthylsulfoxyde, du diméthylformamide et de la pyridine ;
(b) le réactif de déméthylation dans l'étape (1) est le tribormure de bore ou le trichlorure d'aluminium anhydre ;
(c) dans l'étape (1), la température réactionnelle se situe dans la plage de 0 à 30°C quand le tribromure de bore est utilisé comme réactif de déméthylation, et dans la plage de 30 à 200°C quand le trichlorure d'aluminium anhydre est utilisé comme réactif de déméthylation ;
(d) la température pour la réaction d'hydrolyse dans l'étape (3) est de -10 à 40°C, et la durée de la réaction est de 1 à 20 heures ; et
(e) le solvant réactionnel dans l'étape (3) est choisi parmi ou plusieurs de l'acétone, de l'eau, du méthanol, de l'éthanol, du dichlorométhane, du dimétylsulfoxyde, du tétrahydrofuranne ou du dioxanne.

2. Procédé selon la revendication 1, qui est **caractérisé en ce que** l'usage du réactif de déméthylation dans l'étape (1) se situe dans un rapport molaire de 1:1 à 50:1 par rapport au 5,6,7,4'-tétraméthoxy-flavonoïde et la durée de la réaction est de 3 à 20 heures.

3. Procédé selon la revendication 1, qui est **caractérisé en ce que** le produit alcalin utilisé dans l'étape (3) est choisi parmi un ou plusieurs de l'hydroxyde de sodium, de l'hydroxyde de lithium, de l'hydroxyde de potassium, du carbonate de potassium ou du carbonate de sodium, et son usage se situe dans un rapport molaire de 0,1:1 à 50:1 par rapport au composé (II).

4. Procédé selon la revendication 1, qui est **caractérisé en ce que** l'acide utilisé dans l'étape (3) est choisi parmi un ou deux de l'acide sulfurique et de l'acide chlorhydrique, et son usage se situe dans un rapport molaire de 0,1:1 à 50:1 par rapport au composé (II).

5. Procédé selon la revendication 1, qui est caractérisé en que la durée pour le reflux dans l'étape (4) se situe dans la plage de 1 à 10 heures.
